Europäisches Patentamt

European Patent Office

Office européen des brevets

(11) Publication number: **0 382 974**
**A1**

# EUROPEAN PATENT APPLICATION

(21) Application number: 89312619.3

(22) Date of filing: 04.12.89

(51) Int. Cl.⁵: **A61M 25/01**

(30) Priority: 23.01.89 US 300412

(43) Date of publication of application:
22.08.90 Bulletin 90/34

(84) Designated Contracting States:
**BE DE ES FR GB IT NL**

(71) Applicant: **C.R. BARD, INC.**
**730 Central Avenue**
**Murray Hill New Jersey 07974(US)**

(72) Inventor: **MacMore, Bruce**
**224 Main Street**
**South Glen Falls NY 12803(US)**
Inventor: **Stern, David**
**27 Drummer Road Acton**
**Massachusetts 01720(US)**

(74) Representative: **Woodward, John Calvin et al**
**VENNER SHIPLEY & CO. 368 City Road**
**London EC1V 2QA(GB)**

(54) Braided guide wire and method for the use thereof.

(57) An improved guide wire which is steerable, (i.e., torquable without whipping) at lengths of up to approximately 13 ft. while still being flexible enough to negotiate anatomical bends. Also a method of utilizing such guide wire in conjunction with the viewing properties of an endoscope to reach certain gastrointestinal sites, thereby permitting selected medical procedures to be performed.

More particularly, the improved guide wire of this invention has a solid metal core wire with a tapered distal end and a braided mesh applied under tension over the core wire along its entire length. The mesh may be attached to core wire at one or more selected points along the wire, and a coating of a low lubricity material may be applied to the outside of the mesh.

FIG.3

FIG.4

## BRAIDED GUIDE WIRE METHOD FOR THE USE THEREOF

### Field of the Invention

This invention relates to guide wires for use in various medical applications and more particularly to a guide wire which is adapted to be steerable over lengths of up to approximately 13 ft. while still maintaining adequate flexibility. The invention also relates to a method for using the improved guide wire to reach certain gastrointestinal sites.

### Background of the Invention

In modern medicine, guide wires are being increasingly used to reach various remote anatomical sites. Catheters are then passed over the guide wire to such sites and various medical instruments, such as laser bearing optical fibers or dilation balloons, maybe included with or passed through the catheter to permit selected medical procedures to be performed at such sites. Dyes or various medicinal substances may also be applied to the site through the catheter.

In order for a guide wire to be utilized effectively in reaching remote sites, the guide wire must be flexible enough to negotiate sharp bends through body channels while still being stiff enough to transmit torque from the proximal to the distal end to permit steering of the guide wire. To achieve optimum steerability, not only must the wire transmit torque, but it must be stiff enough so as not to take a set, thereby causing whipping of the wire and making the wire difficult to control. Ideally, it should be possible to transmit torque through the wire with equal facility regardless of the direction in which the wire is torqued.

Unfortunately, the criteria for a guide wire stated above are to some extent mutually exclusive. Thus, while stiffness is required in order for the wire to be steerable, if the wire is too stiff it is not able to negotiate the channel bends in the body. While for lengths up to six or eight feet, it may be possible to select guide wires which are sufficiently stiff to transmit torque and be steerable without being too stiff for use, it has not heretofore been possible to design guide wires of greater length which can meet the demanding guide wire criteria indicated above. Even for shorter wire lengths, compromises may sometimes be required in meeting the criteria.

U.S. Patent 4,545,390, entitled "Steerable Guide Wire for Balloon Dilation Procedure" issued October 8, 1985, is an example of a steerable guide wire designed for use in coronary applications requiring a wire approximately 6 ft. in length. Such wires are typically less than 0.02 inches in diameter. However, such wires are not adapted to be steerable at lengths in the 8 to 13 ft. range and would be too stiff in applications where a diameter much in excess of 0.02 inches is required.

For example, a guide wire may be used in certain gastrointestinal applications described herein where an endoscope is passed through the esophagus of the patient until its distal end is adjacent, for example, the bile duct. Utilizing optical properties of the endoscope, a guide wire is fed through a channel in the endoscope, out the distal end thereof and into the bile duct, and is then moved to a desired site, for example near the gall bladder or the pancreas. A catheter may then be fed over the guide wire and various procedures implemented through the catheter. In such application, a guide wire of up to approximately 13 ft. may be required which guide wire has a diameter in the 0.024 to 0.038 inch range. Such a guide wire must be completely steerable (i.e., torquable without whipping) in order to permit entry into the bile duct while still being flexible enough to traverse 90° and greater bends, a 90° bend for example being required on exiting the endoscope.

Since a guide wire capable of meeting these requirements does not currently exist, it has heretofore been the practice to perform this procedure directly with the catheter. However, this is a very difficult and time consuming procedure. Since the longer the procedure takes, the more aggravated the sensitive tissue in the area of the bile duct becomes, thus further complicating the procedure, the procedure is frequently not successful.

### Summary of the Invention

It is therefore a primary object of this invention to provide a steerable, flexible guide wire which remains steerable at lengths of up to approximately 13 ft. while remaining sufficiently flexible for guide wire applications at diameters of up to approximately 0.04 inches.

It is further object of this invention to provide an improved method of performing certain gastrointestinal procedures by use of a guide wire in conjunction with an endoscope.

In accordance with the above, this invention provides a steerable guide wire which has a solid metal core wire with a tapered distal end and a braided mesh applied under tension over the core wire along its entire length. The mesh may be formed of a metal such as stainless steel or of a combination of a high tensile fabric and thin metal wire. The mesh may be attached to the core wire at one or more selected points along the wire, for example by brazing. A coating of a low lubricity material may also be applied to the outside of the mesh.

The invention also includes a method for positioning a guide wire in a small anatomical duct such as a bile duct, which method comprises the steps of moving an endoscope through a body channel such as the esophagus to position the endoscope adjacent the duct; advancing a steerable guide wire such as the guide wire described above through a channel in the endoscope and out an opening in the distal end thereof; viewing the duct through an optical channel in the endoscope; and utilizing the viewing capability of the endoscope to manually manipulate the wire into the duct. The method may also include the step of moving a catheter over the guide wire into the duct.

The foregoing and other objects, features and advantage of the invention will be apparent from the following more particular description of preferred embodiments of the invention as illustrated in the accompanying drawings.

In the Drawings

FIG. 1 is a partially cut-away side view of a guide wire of a first embodiment of the invention.

FIG 2 is a side view of a core wire for use in the guide wire shown in FIG 1.

FIG. 3 is a sectional view taken along the line 3-3 in FIG. 1.

FIG. 4 is a partially cut-away side view of a guide wire of an alternative embodiment of the invention.

FIG. 5 is a diagram illustrating the method of this invention for utilizing the guide wire in conjunction with an endoscope for gastrointestinal applications.

Detailed Description

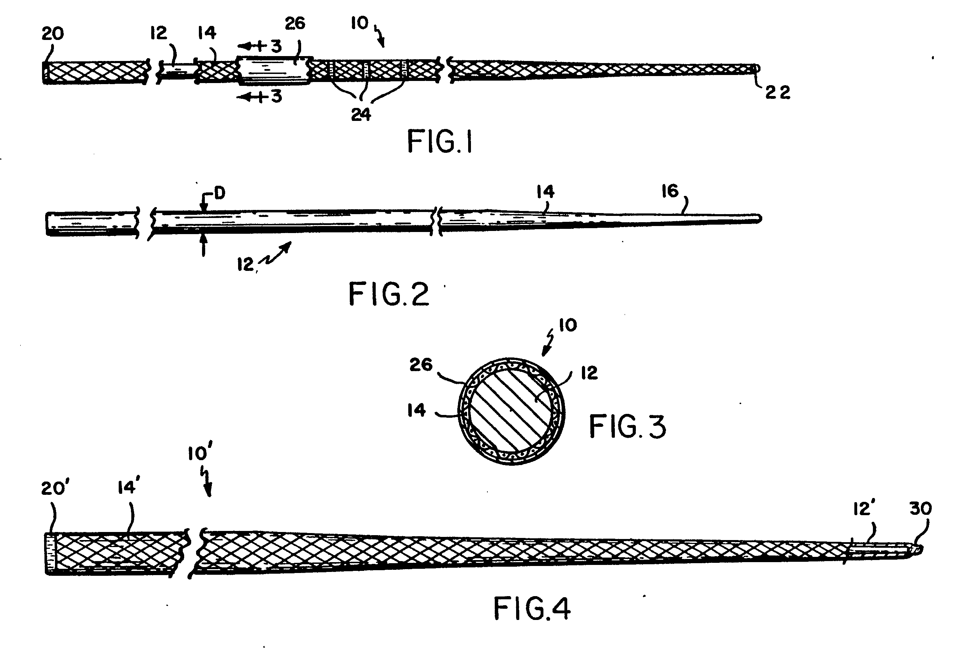

Referring to FIGS. 1-3, it is seen that the guide wire 10 for a first preferred embodiment of the invention has a solid core wire 12 which is covered along its entire length by a braided mesh 14. As may be best seen in FIG. 2, the distal end of core wire 12 is tapered in two sections 16 and 18, the taper covering approximately one foot at the distal end of the wire.

The diameter of the nontapered portions of the core wire will vary with application but will normally be between .021 and .04 inches. Typical widths are in the .024 to .036 inch range. The length of core wire 12 will also vary with application. For certain gastrointestinal procedures to be discussed hereinafter, this length would be the range of approximately 8 ft. to approximately 13 ft; although it should be understood that the teachings of this invention can be advantageously practiced with wires of greater or shorter length. Core wire 12 would typically be formed of stainless steel but could be formed of other biocompatible metals having the requisite torque transmission and flexibility properties.

Braiding 14 may be formed of metal wire, such as stainless steel wire, or may be formed of a combination of high tensile fibers and thin metal wires. Both the tensile fibers and the metal wires would have to be of a material which is biocompatible. Examples of suitable fibers are DacronTM and KevlarTM. The wires would typically be stainless steel.

Braiding 14 is applied to core wire 12 on a standard braiding machine at the highest tension available. The objective with the braiding is to have the braiding grip the wire tight enough during torsion so that there is no slippage between the core wire and braiding but not to have the braiding increase the stiffness of the wire to the point where the guide wire 10 can no longer negotiate body and other bends which it is required to negotiate.

There are basically three factors which can be controlled on the braiding machine which affect torque transmission, whipping, (i.e. control) and stiffness. These factors are the tension under which the braiding is performed, the tent-angle at which the fibers and/or wires are applied to the braiding operation and number of picks-per-inch (i.e. the density) of the braiding weave. The picks-per-inch, in addition to affecting density, also affect the angle of the braiding. The picks-per-inch and tent angle are thus interrelated. The combination of tension, tent angle, and picks-per-inch which will provide desired results for a core wire of given diameter, a particular braiding material, a particular length of guide wire, a particular core wire material and other factors, will, in view of the number of variables involved, need to be empirically determined. Typically, guide wire diameter, length, and the materials for the core wire and the braiding are initially selected based on application and braiding is done at the maximum tension possible for the given braiding machine. Picks-per-inch and tent angle are then adjusted until desired steerability and stiffness are achieved. If, as will be discussed shortly, a coating is applied to the guide wire, this also affects at least stiffness and may require further empirical adjustment in tent angle and picks-per-inch. For an uncoated guide wire approximately 13 feet long having a core wire with an outer diameter of .024" along most of its length, a braiding of 58.7 picks-per-inch has been found to provide suitable results. The picks-per-inch will control the tent angle. This wire might have its diameter ground to 0.015 inches over a 6 inch section 16 and taper to a 0.006 inch diameter over a final 4 inch section 18. The nature of the braiding process is such that the braided wire mesh will always conform to the configuration of the core wire, including, as shown, any tapers in the core wire. If a urethane coating is applied to the wire, the resulting wire may be too stiff with 58.7 picks-per-inch and it may be necessary to reduce the number of picks-per-inch to achieve a desired stiffness. Since the braiding 14 as applied to core wire 12 is symmetric, the steerability of the guide wire 10 is the same regardless of the directions in which it is being torqued.

In order to further secure the braiding 14 to core wire 12, the braiding may be attached to the core wire at suitable points by a suitable means. The preferred means for securing the braiding to the core wire is by brazing. Although soldering, gluing or other means might be employed, these means are not preferred since soldering does not take a coating as well as brazing and gluing is considered to be a slower and generally less effective procedure. In FIG. 1, a braze joint 20 is shown at the proximal end of the guide wire, a braze joint 22 at the distal end, and three braze joints 24 at points along the wire. The exact location of the braze joints 24 is not critical, nor is their spacing. In one embodiment of the invention, the braze joints 24 are approximately 1/3 of the way along the wire from the proximal end and the distance from end to end of the braze joints is approximately 3 inches.

Since the guide wire is to pass through body channels, a catheter, an endoscope channel, or other channels, it is desirable that the wire be coated with a low lubricity coating 26. Since this coating is very thin, it is not easily visible but is shown in FIGS. 1 and 3 of exaggerated width so as to be visible. The coating is preferably a sprayed-on Teflon coating but may also be a coating of a thermoset urethane which may be dipped and/or sprayed on. The coating could also be formed from a continuous sleeve of lubricious material such as Teflon that is formed into a heat shrunk tube that is threaded over the wire. Application of heat causes the Teflon tube or sleeve to shrink and bind itself firmly into the woven braiding 14. As was indicated above, since the coating may affect the stiffness of the guide wire, it will need to be taken into account in selecting various other parameters such as picks-per-inch. However, the coating does not appear to have any noticeable affect on the steerability of the wire.

FIG. 4 illustrate an alternative embodiment of the invention wherein the core wire 12′ tapers uniformly at its distal end 28 rather than in sections. For this embodiment of this invention, only a single braze joint 20′ is provided at the proximal end of the wire. At the distal end, the braiding 14′ extends beyond the end of core wire 12′ and the portion 30 of the braiding extending beyond the end of the core wire is brazed or otherwise secured together to form a soft, slightly flexible tip for the guide wire. This is useful in permitting the wire to get around bends, particularly anatomical bends, and to avoid abrasion or other injury to the walls of such anatomical channels.

While for the guide wire embodiments described above, the braiding is uniform along the entire length of the wire, it may be desirable in some applications to, for example, reduce the picks-per-inch or otherwise change the braiding near the distal end of the wire to reduce stiffness in this area.

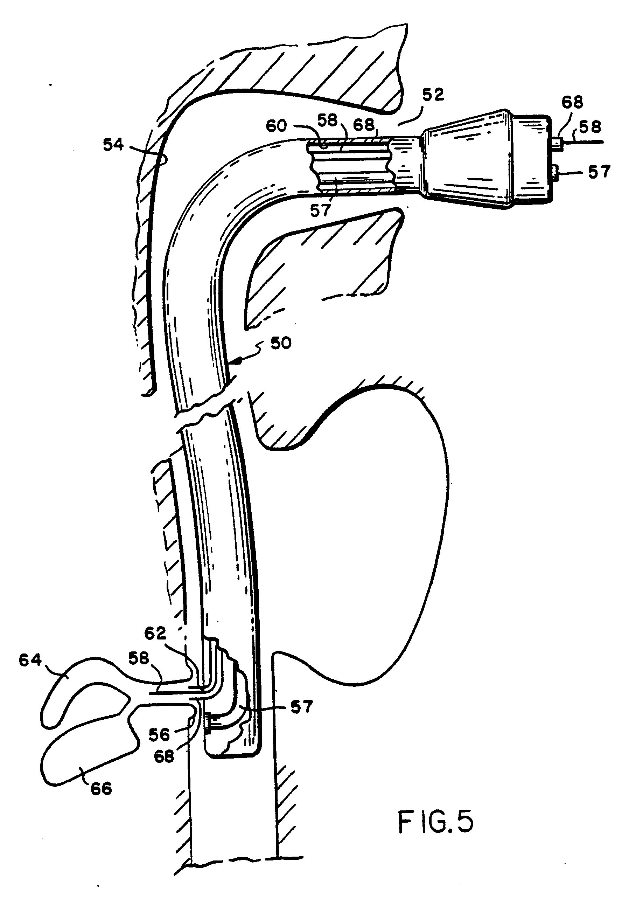

FIG. 5 is a diagram illustrating how the guide wire of this invention might be utilized in conjunction with an endoscope 50 to permit certain gastrointestinal medical procedures to be performed.

The endoscope 50 is positioned through the mouth 52 and esophagus 54 of the patient and through the patient's stomach until the distal end of the endoscope is adjacent to the patient's bile duct 56. A side viewing channel 57, containing for example a fiber optic bundle, is provided in endoscope 50 through which medical personnel may view the progress of the endoscope and which may be utilized to permit the distal end of the endoscope to be properly positioned. A light guide channel is also typically provided in an endoscope to facilitate viewing. A guide wire 58, which may for example be a guide wire of the types described above in conjunction with FIGS. 1-4, is then passed through an open channel 60 in the endoscope and out an opening 62 in the endoscope's distal side. Using optical channel 57, the medical personnel may then position the guide wire 58 in the bile duct 56 and advance the wire through the bile duct to a desired body site. Since guide wire 58 is thick enough to be radiopaque, standard X-ray techniques may be used to view the progress of the guide wire through the bile duct and to advance the guide wire to a desired site. For example, the guide wire may be advanced to or adjacent to the gall bladder 64 or the pancreas 66. Once the guide wire 58 is in the bile duct 56, a catheter 68 may be passed over guide wire 58 into bile duct 56 and used, for example, to pass radiopaque dye into the duct to assist in the advancing of the guide wire to the desired site. This, however, must be done carefully to avoid damage to the pancreas or other organs.

Once the guide wire 58 has been advanced to the desired site, catheter 68 may be advanced over the guide wire to the site. Guide wire 58 may then be removed and either medicinal substances may be delivered to the site through catheter 68 to, for example, break up gall stones or other medical instruments known in the art, such as a balloon catheter, laser optical device or the like, may be brought to the site either as part of the catheter or through the catheter.

A guide wire has thus been provided which exhibits improved steerability even at substantial lengths while still permitting the guide wire to have adequate flexibility. A method for performing various gastrointernal procedures has also been provided which utilizes a guide wire such as the improved guide wire of this invention in conjunction with an endoscope or similar device to reach and permit treatment at various remote gastrointestinal sites.

While the invention has been described above with reference to preferred embodiments, and various modifications to such embodiments have been discussed, it should be understood that the forgoing and other changes in form and detail may be made thereon by one skilled in the art without departing from the spirit and scope of the invention.

## Claims

1. A guide wire comprising:
a solid metal core wire having a tapered distal end; and
a mesh braided under tension over the core wire along its entire length.

2. A guide wire as claimed in claim 1 wherein said braided mesh is formed of thin stainless steel wire.

3. A guide wire as claimed in claim 1 wherein said braided mesh is formed of a combination of high tensile fibers and thin metal wires.

4. A guide wire as claimed in claim 3 wherein the fibers are Dacron fibers and the wires are stainless steel wires.

5. A guide wire as claimed in claim 1 wherein said core wire is formed of stainless steel.

6. A guide wire as claimed in claim 1 including means for attaching the braided mesh to the core wire at at least selected point along the wire.

7. A guide wire as claimed in claim 6 wherein said means for attaching attaches the braided mesh and the core wire at at least the proximal end of the core wire.

8. A guide wire as claimed in claim 7 wherein said means for attaching attaches the braided mesh and the core wire at the ends and at selected intermediate points.

9. A guide wire as claimed in claim 6 wherein said attaching means is operative to braze the mesh and core wire.

10. A guide wire as claimed in claim 6 wherein said attaching means includes means for soldering said braided mesh and core wire.

11. A guide wire as claimed in claim 1 wherein said braided mesh extends slightly beyond the distal end of said core wire; and
including means for securing together the sides of the extended mesh portion to form a flexible distal tip for the guide wire.

12. A guidewire as claimed in claim 11 wherein said means for securing includes means for brazing said sides together.

13. A guide wire as claimed in claim 1 including a coating of a low lubricity material applied to the outside of said braided mesh.

14. A guide wire as claimed in claim 13 wherein said coating is a Teflon coating which is sprayed on.

15. A guide wire as claimed in claim 13 wherein said coating is a heat shrink Teflon tube which is heat shrunk into the braided mesh.

16. A guide wire as claimed in claim 1 wherein the braiding of said mesh is performed with a selected braiding tension, a selected tent angle for the material forming said mesh and a selected picks-per-inch for the braiding; and
wherein said braiding tension, tent angle and picks-per-inch are combined for a given guide wire such that the guide wire is capable of transmitting torque without whipping and with the wire not being too stiff to negotiate right angle bends.

17. A guide wire as claimed in claim 16 wherein the picks-per-inch for the braiding is reduced near the distal end to reduce stiffness.

18. A guide wire as claimed in claim 1 wherein the length of said wire is at least 8 ft.

19. A method for positioning a guide wire in a small anatomical duct comprising the steps of:
moving an endoscope through a body channel to a position adjacent to said duct;
advancing a steerable guide wire through a channel in the endoscope and out an opening in the distal end thereof;
viewing the duct through an optical channel in the endoscope; and
utilizing said viewing step to manipulate said wire into said duct.

20. A method as claimed in claim 19 wherein said body channel includes the esophagus, and wherein said duct is a bile duct.

21. A method as claimed in claim 19 including the step of moving a catheter over said guide wire into said duct.

FIG.1

FIG.2

FIG.3

FIG.4

FIG.5

European Patent Office

**PARTIAL EUROPEAN SEARCH REPORT**
which under Rule 45 of the European Patent Convention
shall be considered, for the purposes of subsequent
proceedings, as the European search report

Application number

EP 89 31 2619

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 132 694 (INTERMEDICAT) <br> * Abstract; figures 1,2 * | 1 | A 61 M 25/01 |
| A | GB-A-2 017 182 (FRESENIUS) <br> * Abstract; figure 2 * | 1 | |
| A | EP-A-0 200 430 (ADVANCED CARDIO-VASCULAR SYSTEMS) | | |
| A,D | GB-A-2 127 294 (C.R. BARD INC.) <br> & US-A-4 545 390 | | |

TECHNICAL FIELDS
SEARCHED (Int. Cl.4)

A 61 M
D 04 C

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-18
Claims searched incompletely:
Claims not searched: 19-21
Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 14-05-1990 | CLARKSON |